(19) Europäisches Patentamt

European Patent Office

Office européen des brevets

(11) **EP 0 542 303 B1**

(12) **EUROPÄISCHE PATENTSCHRIFT**

(45) Veröffentlichungstag und Bekanntmachung des Hinweises auf die Patenterteilung:
**02.02.2000 Patentblatt 2000/05**

(51) Int. Cl.$^7$: **A61K 35/16**

(21) Anmeldenummer: **92119472.6**

(22) Anmeldetag: **13.11.1992**

(54) **Verfahren zur Herstellung eines biologisch aktiven menschlichen Blutserums**

Process for preparing a biologically-active human blood serum

Procédé pour la préparation d'un sérum sanguin humain biologiquement actif

(84) Benannte Vertragsstaaten:
**BE CH DE ES FR GB IT LI**

(30) Priorität: **13.11.1991 SU 5012770**

(43) Veröffentlichungstag der Anmeldung:
**19.05.1993 Patentblatt 1993/20**

(73) Patentinhaber:
**INDIVIDUALNOE CHASTNOE PREDPRIYATIE
SCHESTAKOVA INSTITUT ZHIZNI
Moscow (RU)**

(72) Erfinder:
**Shestakov, Vitaly Alexandrovich
Moskau (SU)**

(74) Vertreter:
**Frank, Veit Peter, Dipl.-Ing. et al
Hoffmann Eitle,
Patent- und Rechtsanwälte
Arabellastrasse 4
81925 München (DE)**

(56) Entgegenhaltungen:
**CH-A- 90 887**

• **CHEMICAL ABSTRACTS, vol. 112, no. 9, 26. Februar 1990, Columbus, Ohio, US; abstract no. 70222, LUNSTRA ET AL 'EFFECTS OF NATURAL MATING STIMULI ON SERUM LUTEINIZING HORMONE,TESTOSTERONE AND 17BETA-ESTRADIOL IN YEARLING BEEF BULLS' Seite 108 ;Spalte 2 ; & J.ANIM.SCI., Bd.67, Nr.12, 1989 Seiten 3277 - 3288**

**Beschreibung**

Die Erfindung betrifft ein Verfahren zur Herstellung eines biologisch aktiven menschlichen Blutserums, welches die Resistenz gegen endogene und exogene Faktoren im menschlichen Empfänger erhöht und sich in der klinischen Medizin zur gezielten Regulation der Funktionen des Organismus, wie Ermüdung, Temperaturempfinden, Schlafbedürfnis und auch Wunsch nach Alkohol oder Nikotin, verwenden läßt.

Weitbekannt sind Verfahren zur Herstellung eines Blutserums, die auf der Spenderblutentnahme, der Inkubation, Trennung und Konservierung des Serums (1,2,3,4) beruhen. Bekannt ist auch ein Verfahren zur Herstellung eines biologisch aktiven Serums (2). Das Verfahren besteht darin, daß man Infektionsagenzien auf den Organismus einwirken läßt, so daß Antikörper im Blut gebildet und dann mit dem Blutserum hergestellt werden. Wir interessieren uns für die Möglichkeit, ein Blutserum herzustellen, welches auf anders an das Immunsystem nicht gebundene Systeme des Organismus wirkt. Es gibt viele exogene und endogene Faktoren im uns umgebenden Leben, die auf den Menschen einen negativen Einfluß ausüben, aber gegen die mittels des oben angegebenen biologisch aktiven Immunserums zu kämpfen unmöglich ist.

Weiterhin ist aus der CH-A-90887 ein Verfahren zur Herstellung eines Blutserums bekannt, bei welchem Tiere durch vitaminreiche Nahrung und ständiges Blutabnehmen angeregt werden, Blut mit erhöhtem Hämoglobin- und Erythrocytengehalt zu bilden. Eine Injektion des aus diesem Blut erhaltenen Serums bei anderen Spezies regt bei diesen die Tätigkeit der blutbildenden Organe an. Auch dieses Serum eignet sich nicht dazu, die Resistenz gegen spezielle endogene und exogene Faktoren im menschlichen Empfänger zu erhöhen.

Die Aufgabe, auf deren Lösung die vorliegende Erfindung gerichtet ist, ist die Herstellung eines biologisch aktiven Blutserums, das die Resistenz gegen endogene und exogene Faktoren, wie Atmosphärendruck, Temperatur, Schwerkraft, Licht, Hunger, Durst, Schlafbedürfnis, Geschlechtsbedürfnis und dergleichen, erhöht.

Zur Lösung der gestellten Aufgabe wird ein neues Verfahren zur Herstellung eines biologisch aktiven Serums vorgeschlagen, weiches darin besteht, daß ein menschlicher Spender vor der Blutentnahme 10 min bis 24 Stunden lang den gewünschten exogenen (Atmosphärendruck, Temperatur, Schwerkraft, Licht u. a. m.) oder endogenen (Hunger, Durst, Schlaf-und Geschlechtsbedürfnis und dgl.) Faktoren ausgesetzt wird.

Durch diese Einwirkung der entsprechenden endogenen oder exogenen Reize wird der menschliche Spender in einen Zustand gebracht, bei dem das aus dem Blut gewonnene Serum spezifische Eigenschaften besitzt. Es liegt also ein technisches Resultat, d. h. ein Serum vor, welches spezifische Eigenschaften besitzt und dabei keine negative Einwirkung auf den Organismus ausübt.

Je nach dar Art der Einwirkung auf den Organismus stellt man Seren her, deren biologische Aktivität von unterschiedchem Charakter ist, und zwar:

    1. myogen
    2. somnogen
    3. ophthalmogen
    4. audioaktiv
    5. thermoaktiv.
    6. diätaktiv
    7. sexaktiv
    8. antihypoxisch
    9. antialkoholisch
    10. antinikotisch

Das Verfahren wird wie folgt durchgeführt.

Nach einer Einwirkung des entsprechenden endogenen oder exogenen Faktors, wie Arbeitbelastung, Schlafentzug, Kälte, Hunger, Übersättigung mit Nahrung, Alkohol oder Nikotin, auf den Organismus, die zeitlich an den jeweiligen Reiz angepaßt ist, entnimmt man das Blut in einer Menge von 70 ml mit Hilfe des Blutentnahmesystems für einmalige Applikation BK 10-01, bringt es in den Behälter "Kompoplast" ein und beläßt es innerhalb eines Kühlers bei 4 °C für 24 Stunden . Dann wird das Serum durch elektrische Absaugung unter sterilen Bedingungen getrennt und in sterile Flaschen zwecks darauffolgender Konservierung in einem Kühlraum bei - 25 °C abgefüllt.

Konkrete Ausführungsbeispiele

Sexaktives Serum

12 freiwillige Studenten der Moskauer Hochschule für Stahl und Legierungen im Alter von 18-25 Jahren, praktisch gesund, mit der medizinischen Grundgruppe haben sich vor der Blutentnahme (am 14. Mai 1991) den Videofilm "Griechischer Feigenbaum" (russischer Filmtitel) innerhalb von 10 bis 15 min angesehen. Das Geschlechtserregungsniveau erreichte dabei 8,36 bis 0,71 Grad (nach einer in 10 Grade eingeteilten Skala). Es wurden von jedem 70 ml Blut mit Hilfe des Blutentnahmesystems BK 10-01 für einmalige Applikation gewonnen. Das Glut wurde dann in den Behälter "Kompoplast" eingebracht und in einem Kühler bei 4 °C für 24 Stunden gehalten. Durch elektrische Absaugung unter sterilen Bedingungen wurde das Serum getrennt, in sterile Flaschen abgefüllt und in einem Kühlraum bei - 25 °C konserviert. Somnogenes Serum

15 freiwillige Studenten der Moskauer Hochschule für Stahl und Legierungen, im Alter von 18-24 Jahren, praktisch gesund, mit der medizinischen Grundgruppe, schliefen vor der Blutentnahme (am 5. September 1991) innerhalb von 24 Stunden nicht. Man führte die Blutentnahme, die Konservierung und Trennung des Serums analog dem vorhergehenden Beispiel durch.

Ophthalmoaktives Serum

10 freiwillige Studenten der Moskauer Hochschule für Stahl und Legierungen, im Alter von 18-25 Jahren, praktisch gesund, mit der medizinischen Grundgruppe befanden sich vor der Blutentnahme (am 18. September)1991) innerhalb von 50 min unter Bedingungen einer geringen Belichtung (0.2 lx), Man führte die Blutentnahme, die Konservierung und Trennung des Serums analog den vorhergehenden Beispielen durch.

Myogenaktives Serum

10 Sportler als Freiwillige im Alter von 18-25 Jahren, praktisch gesund, mit der medizinischen Grundgruppe wurden vor der Blutentnahme einer Arbeitsbelastungsprobe innerhalb von 10 bis 15 min ausgesetzt. Die Blutentnahme, die Konservierung und Trennung des Serums führte man analog den vorhergehenden Beispielen durch.

Audioaktives Serum

12 Studenten als Freiwillige im Alter von 18-22 Jahren, praktisch gesund, mit der medizinischen Grundgruppe, wurden vor der Blutentnahme in einem schallisolierten Zimmer für 45 bis 60 min untergebracht.
Man führte die Blutentnahme, die Konservierung und Trennung analog den vorhergeherden Beispielen durch.

Antihypoxisches Serum

Untersucht wurden 18 Männer im Alter von 18-25 Jahren, bei denen chronische Lungeninsuffizienz diagnostiziert wurde.Das antihypoxische Serum wurde bei Spendern nach ihrem Aufenthalt innerhalb von 30 min in einer Unterdruckkammer bei einem Druck von 666 bis 733 hPa (500 bis 550 Torr) gewonnen. Die Blutentnahme, die Konservierung und Trennung des Serums führte man analog den vorhergehenden Beispielen durch.

Thermoaktives Serum (erhöht die Hitzebeständigkeit)

18 Studenten als Freiwillige im Alter von 18-22 Jahren, praktisch gesund, mit der medizinischen Grundgruppe, wurden vor der Blutentnahme der Einwirkung von hohen Temperaturen (65 bis 67 °C) innerhalb von 45 bis 60 min ausgesetzt. Man führte die Blutentnahme, die Konservierung und Trennung des Serums analog den vorhergehenden Beispielen durch.

Thermoaktives Serum (erhöht die Kältebeständigkeit)

16 Studenten als Freiwillige im Alter von 18-22 Jahren, praktisch gesund, mit der medizinischen Grundgruppe, wurden vor der Blutentnahme der Einwirkung von geringeren Temperaturen (+10 bis +14 °C) innerhalb von 45 bis 60 min ausgesetzt.
Man führte die Blutentnahme, die Konservierung und Trennung des Serums analog den vorhergehen - den Beispielen durch.

Diätaktives Serum (stimuliert den Appetit)

Bei 14 Studenten der Moskauer Hochschule für Stahl und Legierungen als Freiwillige, im Alter von 18-25 Jahren, praktisch gesund, mit der medizinischen Grundgruppe , erfolgte die Deprivation der Nahrung innerhalb von 24 Stunden vor der Blutentnahme (am 4. Juni 1991). Man führte die Blutentnahme, die Konservierung und Trennung des Serums analog den vorhergehenden Beispielen durch.

3

Diätaktives Serum (nimmt das Hungergefühl ab)

12 Männer als Freiwillige im Alter von 25-40 Jahren, praktisch gesund, mit der medizinischen Grundgruppe, erhielten vor der Blutentnahme kalorienreiche Diät (21000 bis 25000 kj (5000 bis 6000 kcal)) innerhalb von 24 Stunden. Man führte die Blutentnahme, die Konservierung und Trennung des Serums analog den vorhergehenden Beispielen durch.

Antialkoholisches Serum

30 Männer als Freiwillige im Alter von 22-50 Jahren, praktisch gesund, mit der medizinischen Grundgruppe, wurden vor der Blutentnahme durch Alkohol (5 bis 8 Stunden) so lange provoziert, bis der volle Widerwillen gegen Spirituosen auftritt. Man führte die Blutentnahme, die Konservierung und Trennung des Serums analog den vorhergehenden Beispielen durch.

Antinikotinisches Serum

18 Studenten als Freiwillige im Altar von 18-22 Jahren, praktisch gesund, mit der medizinischen Grundgruppe, wurden vor der Blutentnahme durch Nikotin (20 bis 40 min) bis zum Auftreten dem Gefühls der starken Abneigung gegen Rauchen provoziert.

Man führte die Blutentnahme, die Konservierung und Trennung des Serums analog den vorhergehenden Beispielen durch.

Beispiele, welche die Wirksamkeit der hergestellten Seren erläutern

Sexaktives Serum

Das sexaktive Serum wird bei Störungen des sexuellen Verhaltens für Prophylaxe und Behandlung des geschlechtlichen Unvermögens und Verlängerung der geschlechtlichen Langlebigkeit verwendet.

Die positive Wirkung durch Applikation des sexaktiven Serums tritt 10 bis 30 min nach der einmaligen Einnahme in Erscheinung. Die Heilwirkung einer Dosis dauert mindestens 2 Tage. Durch Verwendung des sexaktiven Serums bedingte Gewöhnung und Nebenwirkungen sind nicht festgestellt.

Beispiel. Untersucht sind 12 Männer im Alter von 40-50 Jahren mit Depression der Geschlechtserregung. Des sexaktive Serum wurde in einer Dosis von 20 ml intramuskulär eingeführt. 6 Stunden nach dem Injektion stieg die Geschlechtserregung von $4,50 \pm 0,3$ bis $7,83 \pm 0,5$ Grade nach der 10-Grad-Skala an, während der Tonus des Zeugungsglieds von $33,3 \pm 1,6$ bis $57,9 \pm 1,9$ mt (bezogen auf glas = 100mt, Myotonometer von WNIITK-SSSR)

erhöht wurde. Zubereitungsformen des sexaktiven Serums: Lösungen, Pulver, Kapseln, Rektalsuppositorien.

Die Aufbewahrungsfrist von Fertigformen bei + 4 °C erreicht 3 Jahre.

Somnogenes Serum

Des somnogene Serum verwendet man zu Zwecken der Prophylaxe und Behandlung von Schlafstörungen und psychoemotionalen Stressen, es beseitigt die Schlaflosigkeit und organisiert die Struktur des Normalschlafs.

Die positive Wirkung tritt 15 bis 30 min nach der einmaligen Einnahme des somnogenen Serums in Erscheinung.

Durch Verwendung des somnogenen Serums bedingte Gewöhnung und Nebenwirkungen sind nicht festgestellt.

Beispiele. Bei 15 Studenten als Freiwilligen zeigten sich vor der Prüfungstagung für die geistige Übermüdung charakteristische Unruhe, Aufregung, Unsicherheit und Schlafstörungen. Die intramuskuläre Injektion des somnogenen Serums in einer Dosis von 20 ml bewirkte die Steigerung des Leistungsspektrums der Delta - und Thetaaktivität, des Auftreten von "Schlafspindeln" im Betabereich des EEG's und den stabilen Wunsch zu schlafen: 10-Grad-Skale "Wachen-Schlaf" gibt die Änderung von $3,14 \pm 0,1$ bis $8,67 \pm 0.4$ Grade an. 40 min nach der Applikation des somnogenen Serums schliefen alle Freiwilligen, wobei des EEG-Bild des Normalschlafs typisch war. Der Schlaf war tief und erfrischend. Die geistige Arbeitsfähigkeit am nächsten Tag nach der Korrektion durch somnogenes Serum war hoch.

Zubereitungsformen des somnogenen Serums: Lösungen, Pulver, Kapseln, Rektalsuppositorien.

Die Aufbewahrungsfrist von Fertigformen bei +4°C erreicht 3 Jahre.

Ophthalmoaktives Serum

Das ophthalmoaktive Serum benutzt man bei der konservativen Behandlung von Augenkrankheiten, nämlich diabetische Retinopathie und ihre Komplikationen, Glaukom, Myopie u.a.

Bei der Applikation des ophthalmoaktiven Serums stellt man höhere Sehfunktionen, längere Heilwirkung fest. Das ophthalmoaktivs Serum wird in einer Dosis von 0,15 ml/kg Körpergewicht einmal täglich 3 Tage lang intramuskulär injiziert. Die positive Wirkung des Serums wird innerhalb von 2 bis 3 Monate konstantgehalten.

Komplikationen seitens anderer Organs und Systems und Nebenwirkungen stellt man nicht fest.

Beispiel 1. Der Kranke K., 58 Jahre alt. Diagnose:präproliferative diabetische Retinopathie beider Augen, rezidivierender Hämophthalmus des linken Auges. Nebenkrankheiten: mittelschwerer Diabetes mellitus, Hochdruckerkrankung, diabetische Nephropathie.

Ursprüngliche Sehschärfe: das rechte Auge 0,2, nicht korrigiert, des links Auge 0,1, nicht korrigiert.

Der objektive Zustand des rechten Auges: Optikusscheibe ist blässlich, Grenzen sind scharf, Venen vollblütig, Arterien verengt, stark skleriert, eine Vielzahl von trockenen und feuchten Exsudaten (im makulären Gebiet ein hartes Exsudat mit einem 2 bis 3 Durchmesser des Optikusscheibe betragenden Durchmesser), paramakulär und an der Peripherie des Augenhintergrunds viele präretinale und retinals Hämorrhagien unterschiedlicher Form und Größe, Bereiche der retinalen Neovaskularisation. Das links Auge: der vom Augenhintergrund ausgehende Reflex ist dunkelrot wegen des fast vollständigen Hämophthalmus.

Das ophthalmoaktivs Serum wurde intramuskulär täglich als 3 Injektionen je Kur in einer Menge von 12,6 ml (ausgehend von 0,15 ml/kg Körpergewicht bei einem Korpergewicht von 84 kg) eingeführt. 1 Woche nach der Beendigung dar Kur nahm die Sehschärfe beider Augen zu, nämlich links um 10 % und rechts um 5 %, der Patient bemerkte die Verbesserung des Allgemeinzustands und die Erhöhung der Arbeitsfühigksit 2 Wochen nach der durchgeführten Kur stieg die Sehschärfe des rechten Auges um 15 % und die des linken um 20 % gegenüber der ursprünglichan an. 1 Monat nach der Beendigung der Kur war Sehschärfe des rechten Auges 0,4 gleich (sie erhöhte sich gegenüber der Ausgangsgröße um 30 bis 40 % und betrug 0,4 bis 0,5). Am rechten Auge entgingen in bedeutendem Maße die retinalen und präretinalen Hämorrhagien, die exsudativen Herde im makulären und paramakulären Gebiet verminderten sich im Durchmesser um des 1,5 - bis 2fache, am linken Auge entging die Blutung in den Glaskörper völlig, der Augenhintergrund wurde der Betrachtung und der weiteren Behandlung zugänglich. Bei der nach 3 Monaten vorgenommenen Kontrolluntersuchung war eine Verschlechterung des Augenzustande und des Allgemeinbefindens nicht zu konstatieren. Man registrierte keine Nebenwirkungen und Komplikationen sowohl bei der Einführung des Serums als auch in der Folgezeit.

Beispiel 2. Die Kranke K., 62 Jahrs alt. Diagnose; operiertes entwickeltes Glaukom, Nebenkrankheiten: Kardiosklerose nach dem Infarkt, Hochdruckerkrankung IIB, mittelschwerer Diabetes mellitus. An dem linken früher anläßlich des Glaukoms operierten Auge wurde eine Erhöhung des intraokularen Druckes von 22,7 auf 37,3 bis 40,0 h Pa (von 17,0 auf 28,0 bis 30,0 mm Hg-Säule) registriert, die Sehfelder wurden stark verengt;von 30-45-55 bis 10-15-20 vom Fixierpunkt aus. Objektiv: Optikusscheibe ist blässlich, Grenzen sind scharf, glaukomatöse Exkavation, Stauungsvenen, Arterien verengt, skleriert. Trotz der Kompensation des intraokularen Druckes durch Wahl von Myotika mit dem Ziel, die weitere Verengung der Sehfelder und den Verlust der Restfunktionen zu verhindern, beschloß man, die Kranke wiederholt zu operieren. ES wurde eine Kur mit dem ophthalmoaktiven Serum vorher gemacht. Das ophthalmoaktive Serum wurde intramuskulär täglich je 11,7 ml innerhalb von 3 Tagen je Kur (ausgehend von 0,15 ml/kg Körpergewicht bei einem Gesamtgewicht von 78 kg) injiziert, 2 Wochen nach der Serumbehandlung wurde die Erweiterung der Sehfelder auf 20-30-45 im Vergleich zur vorigen Untersuchung konstatiert. Dieser Zustand wurde nachfolgend konstantgehalten, was im Laufe der Untersuchungen nach 2 und 3 Monaten festgestellt wurde. Bei der Betrachtung des Augenhintergrunds wurde eine geringere Dekolorierung der Optikusscheibe und ein geringerar Krampf der arteriellen Gefäße bemerkt. Im Zusammenhang mit diesen günstigen Ergebnissen der Verwendung des ophthalmoaktiven Serums wurde die wiederholte antiglaukomatöse Operation aufgehoben.

Beispiel 3. Die Kranke, 17 Jahre alt. Diagnose: mittelschwere Myopie, Akkomodationskrampf. Nebenkrankheiten: Bronchialasthma, verbunden mit der allergischen Komponente. Sehschärfe; 0,2 s - 3,5 D = 0,7-0,8 rechts, 0,1 s - 4,5 D = 0,7-0,8 links.

Des ophthalmoaktive Serum wurde täglich innerhalb von 3 Tagen in einer Dosis von 9,15 ml ausgehend von 0,15 ml/kg Körpergewicht bei einem Körpergewicht von 61 kg intramuskulär injiziert. 10 Tage nach dem Kuranfang war die Erhöhung der Sehschärfe bis 0,2 s - 2,0 D = 0,9 am rechten Auge, 0,3 s - 3,0 D = 0,9 - 1.0 am linken Auge registriert.

Bei der nach 2 Wochen, 1 und 2 Monaten durchgeführten Untersuchung blieben die Sehfunktionen auf hoher Ebene bei einer ausreichend verminderten Korrektion erhalten (gegenüber den Ausgangsdaten).

Zubereitungsformen: Lösungen, Pulver.

Die Aufbewahrungsfrist von Feltigformen bei +4 °C erreicht 3 Jahre.

EP 0 542 303 B1

Myogenes Serum

Man verwendet das myogene Serum Zwecks Erhöhung der allgemeinen und speziellen körperlichen Arbeitsfähigkeit, Vorbeugung und Behandlung von asthenischen Zuständen, Immunitätsdepressionen, der geistigen und körperlichen Übermüdung.

Die positive Wirkung tritt 60 min nach der einmaligen Einnahme des myogenen Serums in Erscheinung.

Durch Verwendung des myogenen Serums bedingte Gewöhnung und Nebenwirkungen sind nicht festgestellt.

Beispiel 1. Bei der Untersuchung der Wirkung des myogenen Serums bestand eine Versuchsgruppe aus Sportiern und zwar Leichtathleten, die sich auf 400 und 800 m langen Flachlaufstrecken spezialisieren (28.12.91). Das Altar der Sportler lag zwischen 16 und 18 Jahren. Es gab 4 Meister des Sports, und 6 Menschen waren Sportler der Meisterklasse. Das Gewicht der Sportler betrug 63 bis 71 kg und ihre Höhe 175 bis 183 cm.

Zur Kontrollgruppe gehörten Sportler der gleichen Sportdisziplin im Altar von 16 bis 18 Jahren, darunter 5 Meister des Sports und 5 Menschen der Meisterklasse. Das Gewicht der Sportler der Kontrollgruppebetrug 63 bis 70 kg und ihre Höhe 174 bis 181 cm.

Bei den Sportlern der Versuchsgruppe wurde 2 Stunden nach dar enteralen Applikation des myogenen Serums in einer Dosis von 0.3 ml/kg Körpergewicht eine Erhöhung der körperlichen Arbeitsfähigkeit um 12,6 % (p $C_{170/kg\ Körpergewicht}$),bezogen auf den ursprünglichen Hintergrund, und um 12,8 % gegenüber der Kontrollgruppe bemerkt.

Die Versuchsgruppe leistete ein größeres Arbeitsvolumen bei einer geringeren Herzfrequenz unter intensiverer Wiederkehr der Herzfrequenz zur dritten Minute nach der körperlichen Belastung mit einem Fahrradergometer.

Pathologische Änderungen seitens des Herz- und Kreislaufsystems und des Blutes wurden nicht registriert.

Am zweiten Tag nach der einmaligen Einnahme des myogenen Serums blieb die hohe körperliche Leistungsfäigkeit bei der Versuchsgruppe erhalten.

Am fünften Tag kam die körperliche Leistungsfähigkeit der Sportler der Versuchsgruppe an die Ausgangshöhe zurück.

Beispiel 2. Das myogene Serum wurde intramuskulär und enteral bei der Vorbereitung von 24 Boxern der UdSSR zur Beteiligung an den Spielen guten Willens in der Stadt Seattle (1990) appliziert. Was das Auftreten der Boxer anbetrifft, so sagten die Fachleute (ohne Berücksichtigung der Applikation eines myogenen Serums) 9 Medaillen in beliebigem Wert vorher, tatsächlich wurden 20 Medaillen gewonnen.

Die dreimalige Doping-Kontrolle der Boxer ergab eine negative Reaktion.

Gesamteinschätzung von Trainern und Mannschaftsarzt: die Verwendung des myogenen Serums wirkt positiv auf die Widerstandskraft und die körperliche Leistungsfähigkeit von Boxern ein. Subjektive Einschätzung der meisten Boxer: die Verwendung des myogenen Serums begünstigt das erfolgreiche Auftreten, und es ist bei der Verbereitung von Sportlern zu verantwortlichen Wettkämpfen zweckmäßigerweise zu applizieren.

Beispiel 3. Das myogene Serum wurde bei 50 Männern im Alter von 20 bis 25 Jahren verwendet, die keinen Sport betrieben.

Die intramuskuläre Einführung des myogenen Serums in einer Dosis von 0,3 ml/kg Körpergewicht bewirkte eine Erhöhung der allgemeinen körperlichen Arbeitsfähigkeit um 12 % (p $C_{170}$), der geistigen Arbeitsfähigkeit um 16 % (einfache und komplexe Analoga), Beschleunigung des psychischen Tempos um 19 %, Vergrößerung das Volumens des kurzzeitigen Gedächtnisses um 7 %, Verbesserung des Befindens und Steigerung des Lebenstonus.

Gleichzeitig nahm die Aktivität des Immunsystems zu; das Niveau von Immunglobulinen (Gr. A,M,O) stieg um 26,8 bis 33,3 % an.

Zubereitungsformen des myogenen Serums: Lösungen, Pulver, Kapseln.

Die Aufbewahrungsfrist von Fertigformen des myogenen Serums, d.h. von Pulvern, Kapseln erreicht 5 Jahre und die von Lösungen etwa 1 Jahr bei 10 bis 15 °C.

Audioaktives Serum

Das audioaktive Serum wird zwecks Verbesserung der Gehörschäfe im ganzen Frequenzbereich sowie zur Vorbeugung und Behandlung der Störungen der Gehörschärfe unterschiedlicher Ätiologie verwendet.

Die positive Wirkung des audioaktiven Serums tritt 15 bin 30 min nach der einmaligen Einnahme in Erscheinung. Die Dauer der Heilwirkung bei des dreitägigen Kur beträgt mindestens 5 Wochen.

Gewöhnung und Nebenwirkungen bei andauernder Applikation des audioaktiven Serums sind nicht registriert.

Beispiel. Der Kranke G., 30 Jahre alt, Beschwerde über die Gehörverschlechterung beider Ohren, innerhalb von 5 Jahren der Kranke bringt seine Erkrankung mit der Lärmeinwirkung des Orchesters im Restaurant in Verbindung, wo er innerhalb von mehreren Jahren als Kellner tätig war, war ambulant in ärztlicher Behandlung ohne Erfolg (medikamentöse Therapie, Physiotherapie). Objektiv: das rechte Ohr: Trommelfell von grauer Farbe, mit Erkennungszeichen, Flüstersprache in 1,5 m Entfernung. Umgangssprache in 5 m Entfernung. Das linke Ohr: Trommelfell von grauer Farbe, wird konturiert, Flüstersprache in 1,5 m Entfernung, Umgangssprache in 5 m Entfernung, andere HNO-Organe ohne sichtbare Pathologie. Am 18., 19., 20. November 1986 wurden intramuskuläre Injektionen des audioaktiven Serums in einer Menge von 0,3 ml je 1 kg Körpergewicht in den oberen Außenviertelkreis des Gesäßmuskels gemacht. Subjektiv wurde die Verbesserung der Gehörschärfe beider Ohren, eine bessere Verständlichkeit und größere Deutlichkeit der Sprache sowie die Steigerung der geistigen und körperlichen Arbeitsfähigkeit bemerkt. Nebenwirkungen, hervorgerufen durch das Präparat, fehlten.

Die nachstehende Tabelle zeigt Audiogramme des Kranken G. mit chronischer neurosensorischer Schwerhörigkeit vor Einführung des audioaktiven Serums und zu verschiedener Zeit nach seiner Einführung (das rechte Ohr, Luftleitung).

Wie aus der Tabelle zu ersehen ist, ergab sich nach den Injektionen des audioaktiven Serums eine Erhöhung der Gehörschärfe beim Kranken G. im ganzen audiometrischen Frequenzbereich um 10 bis 30 dB am höchster am Tag der 3. Injektion. Die Gehörschärfe war erhöht, set te sich innerhalb von 5 wochen (Datum der letzten Untersuchung) allmählich herab, wobei sie das Ausgangsniveau sicher Überstieg.

Tabelle

| Hörschwelle vor Injektionen in $d_B$ Schallfrequenz in Hz | 10 | 15 | 10 | 20 | 25 | 35 | 50 |
|---|---|---|---|---|---|---|---|
| Erhöhung der Gehörschärfe in $d_B$ gegenüber Hörschwelle | 125 | 250 | 500 | 1000 | 2000 | 4000 | 8000 |
| am Tag der 3. Injektion | 10 | 15 | 10 | 15 | 15 | 25 | 30 |
| am 5. Tag nach der Injektion | 10 | 15 | 10 | 10 | 15 | 20 | 25 |
| 3 Wochen nach der Injektion | 5 | 10 | 10 | 10 | 10 | 20 | 20 |
| 5 Wochen nach der Injektion | 5 | 5 | 10 | 10 | 5 | 15 | 20 |

Die gleichen Ergebnisse wurden auch am linken Ohr gewonnen.

Zubereitungsformen: Lösungen, Pulver, Rektalsuppositorien.

Die Aufbewahrungsfrist von Fertigformen bei + 4 °C erreicht 3 Jahre.

Antihypoxisches Serum

Das antihypoxische Serum wird zur Vorbeugung von Hypoxieerscheinungen verwendet, vergrößert den Sauerstoffverbrauch, erhöht die körperliche Liestungsfähigkeit.

Die positive Wirkung des antihypoxischen Serums tritt nach 3tätiger Injektionskur in Erscheinung.

Gewöhnung und Nebenwirkungen bei der Applikation des antihypoxischen Serums sind nicht festgestellt.

Beispiel. An 14 Freiwilligen im Alter von 25-45 Jahren, die an der Lungeninsuffizienz leiden, wurde das antihypoxische Serum in einer Dosis von 0,3 mg/kg Körpergewicht innerhalb von 3 Tagen intramuskulär eingeführt.

Nach der Beendigung der Serumtherapie wurde bei den Kranken die positive Dynamik von Kennwerten der äußeren Atmung, des Blutes und der körperlichen Leistungsfähigkeit beobachtet. Die Atemnot verschwand, die Atmungsfrequenz nahm um 25 % ab, die Herzfrequenz verkürzte sich um 8 bis 10 %, der maximale Sauerstoffverbrauch nahm um 15 % zu und die körperliche Leistungsfähigkeit stieg nach den Fahrrad - Ergometrieangaben um 12 % an.

Zubereitungsformen des antihypoxischen Serums: Lösungen, Pulver, Suppositorien. Die Aufbewahrungsfrist von Fertigformen erreicht bei 15 bis 20 °C (Lösungen) etwa 3 Jahre, bei + 4 °C (Pulver, Suppositorien) etwa 5 Jahre.

Thermoaktives Serum (TC-12)

Das thermoaktive Serum TC-12 wird zur Prophylaxe von Erkrankungen, Erhöhung der Stabilität und Beschleunigung der Adaptation an die Einwirkung von Umwelthochtemperaturen verwendet.

Die positive Wirkung tritt 15 bis 30 min nach der einmaligen Einnahme in Erscheinung. Die Dauer der Heilwirkung einer Dosis beträgt mindestens 2 Tage.

Gewöhnung und Nebenwirkung bei der Applikation des TC-12 sind nicht festgestellt.

Beispiel. An 18 Stundenten als Freiwilligen im Alter von 18-22 Jahren, die Umwelthochtemperaturen schwer vertragen, wurde das Serum TC-12 in einer Dosis von 0,3 ml/kg Körpergewicht intramuskulär injiziert. Nach der Applikation von TC-12 befanden sich die Probanden in einer hyperthermischen Kammer bei einer Temperatur von + 70±5 °C und relativen Feuchtigkeit vor 30 bis 40 %. Beim Aufenthalt in der hyperthermischen Kammer wurden die Probanden unter ständige medizinische Beobachtung gestellt, wobei Herzfrequenz. Ekg. Atmungsfrequenz, Sehen, Gehör, Schweißsekretionsintensität usw. registriert wurden.

Die Aufenthaltsdauer unter Hyperthermiebedingungen vor dem Hintergrund der Applikation von TC-12 war auf das 2,5fache länger als ohne Verwendung von TC-12 (Kontrolluntersuchungen der gleichen Probanden).

Zubereitungsformen von TC-12: Lösungen, Pulver, Suppositorien. Die Aufbewahrungsfrist von Fertigformen erreicht bei einer Temperatur von 15 bis 20 °C (Lösungen) etwa 3 Jahre, bei +4 °C (Pulver, Suppositorien) etwa 5 Jahre.

Thermoaktives Serum (TC-7o)

Das thermoaktive Serum TC-70 wird zur Prophylaxe von durch Unterkühlung bedingten Erkrankungen, Erhöhung der Stabilität des Organismus gegen Einwirkung von Tieftemperaturen, Beschleunigung der Adaptation des Menschen an Hypothermie verwendet.

Die positive Wirkung tritt 15 bis 30 min nach der einmaligen Einnahme in Erscheinung. Die Dauer der Heilwirkung einer Dosis beträgt mindestens 2 Tage.

Gewöhnung und Nebenwirkungen bei der Applikation von TC-70 sind nicht festgestellt.

Beispiel. Art 16 Studenten als Freiwilligen im Alter von 18-22 Jahren, die in der kalten Jahreszeit 1990-1991 für Erkältungen oft anfällig waren, wurde das Serum TC-70 in einer Dosis von 0,3 ml/kg Körpergewicht intramuskulär injiziert. 30 min nach der Injektion von TC-70 beobachtete man bei den Probanden eine schnellere Wiederkehr der Temperatur der Hautdecken nach der Kältebelastung nach Marschak-Methode. Im Bereich des Unterarmes und des Schenkels war diese Temperaturwiederkehr um mehr als auf das 2fache intensiver, als vor Applikation von TC-70. Die Zahl der funktionierenden Kältepunkte vor dem Hintergrund der Applikation von TC-70 nahm um 7 % ab. Die Konzentration von Immunglobulinen im Blut erhöhte sich um 15 bis 20 %. Innerhalb von 60 Tagen der medizinischen Beobachtungen waren keine Erkältungskrankheiten registriert.

Zubereitungsformen von TC-70: Lösungen, Pulver, Suppositorien. Die Aufbewahrungsfrist von Fertigformen beträgt bei einer Temperatur von - 15 bis -20 °C (Lösungen) etwa 3 Jahre und bei + 4 °C (Pulver, Suppositorien) etwa 5 Jahre.

Diätaktives Serum

$$\text{(Д,С-Г)}$$

Das diätaktive Serum

DC-Γ

wird zur Vorbeugung und Behandlung von Stoffwechselstörungen bei Asthenie und Depression, Stimulation des Hungergefühls und Erhöhung der Geschmacksempfindlichkeit eingesetzt und begünstigt die Reinigung des Organismus von metabolischen Produkten und steigert die körperliche Leistungsfähigkeit.

Die positive Wirkung von tritt 60 min nach der einmaligen Einnahme in Erscheinung. Die Dauer der Heilwirkung einer Heildosis beträgt mindestens 1 bis 2 Tage.

Gewöhnung und Nebenwirkungen bei der Applikation von

DC-Γ

sind nicht festgestellt.

Beispiel. Untersucht wurden 14 Männer im Alter von 18-25 Jahren mit der Depression von Nahrungsmotivation.

DC-Γ

wurde in einer Dosis von 0,3 ml/kg Körpergewicht intramuskulär injiziert. 45 bis 60 min nach der Injektion nahm das Appetitniveau von 4,01±0,07 auf 8,35±0,09 nach der 10-Grad-Skale "satt-hungrig" zu, die Bewegungsaktivität steigerte sich um 35,57 %, die Zeit der komplizierten sensomotorischen Reaktion verkürzte sich, die körperliche Gesamtleistungsfühigkeit stieg um 4,5 % an.

Zubereitungsformen von

DC-Γ:

Lösungen, Suppositorien.

Die Aufbewahrungsfrist von Fertigformen

DC-Γ :

bei -25 °C beträgt etwa 5 Jahre (Lösungen), bei + 4 °C etwa 1 Jahr (Suppositorien).

Diätaktives Serum (DC-H)

DC-H

ist zur Prophylaxe und Behandlung von Stoffwechselstörungen bei erhöhtem Appetit und ständigem Durst indiziert. Es ist ebenfalls bei der Diätverpflegung und künstlichen Körperpergewichstabnahme angezeigt.

Die positive Wirkung von

DC-H

tritt 60 min nach der einmaligen Einnahme in Erscheinung. Die Dauer der Heilwirkung einer Dosis beträgt mindestens 1 bis 2 Tage.

Beispiel. Untersucht wurden 12 Männer im Alter von 25-40 Jahren mit ständigem Hungergefühl und erhöhtem Körpergewicht.

DC-H

wurde in einer Dosis von 0,3 ml/kg Körpergewicht intramuskulär appliziert. 60 min nach der Injektion von

DC-H

beobachtete man bei Kranken eine Appetitabnahme um das 2,3fache nach der 10-Grad-Skala.

Zubereitungsformen von

DC-H :

Lösungen, Suppositorien.

Die Aufbewahrungsfrist bei -25 °C beträgt etwa 5 Jahre (Lösungen), bei + 4 °C etwa 1 Jahr (Suppositorien).

Antialkoholisches Serum

Das antialkoholische Serum wird zur Prophylaxe und Behandlung des Alkoholismus von unterschiedlichem Grad und um Beseitigung des Alkoholbedarfs beim Menschen verwendet.

Die positive Wirkung tritt 30 bis 60 min nach der einmaligen Einnahme des antialkoholischen Serums in Erscheinung. Die Dauer der Heilwirkung einer Dosis beträgt 2 bis 4 Tage.

Gewöhnung und Nebenwirkungen bei der Applikation des antialkoholischen Serums sind nicht festgestellt.

Beispiel. An 30 Männern im Alter von 22-50 Jahren, die an chronischem Alkoholismus leiden, wurde das antialkoholische Serum täglich in einer Dosis von 0,3 ml/kg Körpergewicht innerhalb von 3 Tagen injiziert. Nach der Verwendung des antialkoholischen Serums beobachtete man bei Kranken im Falls der Provokation des Alkoholismus einen stabilen Brechreflex und keine alkoholische Motivation. Der Brechreflex zeigte sich auch 30 bis 40 Tage nach der Applikation des antialkoholischen Serums, die antialkoholische Motivation blieb ebenfalls erhalten.

Zubereitungsformen des antialkoholischen Serums: Lösungen, Pulver, Suppositorien.

Die Aufbewahrungsfrist von Fertigformen beträgt bei einer zwischen -15 und - 20 °C liegenden Temperatur (Lösungen) etwa 3 Jahre, bei + 4 °C (Pulver) etwa 5 Jahre.

Antinikotinisches Serum

Das antinikotinische Serum wird zur Prophylaxe und Behandlung des Nikotinismus bei Kindern und Erwachsenen, Beseitigung des Nikotinbedarfs des Menschen verwendet.

Die positive Wirkung tritt 3 Tage nach der dreimaligen täglichen Einnahme des antinikotinischen Serums in Erscheinung. Die Dauer der Heilwirkung der kurmäßigen Einnahme des antinikotinischen Serums beträgt 30 bis 40 Tage.

Gewöhnung und Nebenwirkungen bei der Applikation des antinikotinischen Serums sind nicht festgestellt.

Beispiel. An 18 Studenten als Freiwilligen, welche am Nikotinbedarf leiden (etwa 40 Zigaretten täglich rauchen), wurde das antinikotinische Serum in einer Dosis von 0,3 ml/kg Körpergewicht täglich innerhalb von 3 Tagen inframuskulär injiziert. Nach der vorgenommenen Antinikotinkur beobachtete man bei den untersuchten Studenten kein Bedürfnis nach dem Rauchen von Tabak. Im Laufe der 30tägigen klinischen Beobachtung nahmen nur 3 von 18 Studenten das Rauchen wieder auf. Die Wiederaufnahme des Rauchens erklärten sie mit ihrer übermäßigen Streßspannung, hervorgerufen durch Vereinigung des Tagesunterrichts an der Hochschule und der Abendarbeit in einer Baugenossenschaft. 15 andere Studenten haben diese schlechte Angewohnheit aufgegeben.

Zubereitungsformen des antinikotinischen Serums: Lösungen, Pulver und Suppositorien. Die Aufbewahrungsfrist von Fertigformen beträgt bei einer zwischen -15 und -20 °C liegenden Temperatur (Lösungen) etwa 3 Jahre und bei +4 °C (Pulver) etwa 5 Jahre.

| Vergleichspräparate für biologisch aktive Blutseren | | Präparate |
|---|---|---|
| Blutseren | | Präparate |
| 1. | myogenaktiv | Retabolyl, Koffein, Ephedrin, Phenamin |
| 2. | somnogen | Natriumoxybutyrat |
| 3. | sexaktiv | Pantocrin |
| 4. | ophthalmoaktiv | Trental |
| 5. | audioaktiv | Trental |
| 6. | antihypoxisch | Vitamin C, Gutimin |
| 7. | thermoaktiv | |
| | a. erhöht die Hitzebeständigkeit (TC-12) | Acephen, Eleutherococcus |
| | b. erhöht die Kältebeständigkeit (TC-70) | Eleutherococcus |
| 8. | nahrungsmotivationsregelnd | |
| | a. sattheitsstimulierend, apetithemmend (DC-H) | Phepranon, Mephomen, Phenamin |
| | b. appetitanregend (DC-Γ) | Aloe, Insulin, Aufgüsse, Abkochungen |
| 9. | antialkoholisch | Teturam, Inmipar, |
| 10. | antinikotinisch | Citizin, Lobelin |

Alle Arten von Seren weisen gegenüber den traditionellen Analoga keine Nebenwirkungen oder Gegenanzeigen bei der Anwendung auf.

Literaturliste

1. Ivanov, L.V., Svirkovskaja E.L., "Die Organisation großer Kontingente an kostenlosen Immunblutspenden", aus: "Aktuelle Probleme des Transfusionswesens", Moskau, 1983, S. 34-37.

2. Rainer T.I., Rainer I.I. "Antiendotoxische Hyperimmunseren. Einige Grundlagen zu ihrer Herstellung", aus: "Thesenpapiere der Abschlußkonferenz des wissenschaftlichen Forschungsinstituts für Impfstoffe und Seren in Perm", Perm, 1985, S. 12-15.

3. Filatov A.N. "Herstellung und Verwendung von sterilisierten Homo- und Heteroseren." Arbeiten des Leningrader wissenschaftlichen Forschungsinstituts für Bluttransfusion, 1943, Band 5, S. 130-136.

4. Ščesjuk D.A. "Der Einfluß von Immunseren auf einige Parameter der Reaktionsfähigkeit des Organismus", aus: "Probleme der Patologie in Versuchen und in der Klinik", L'vov, 1980, Band 4, S. 111-112.

## Patentansprüche

**1.** Verfahren zur Herstellung eines biologisch aktiven menschlichen Blutserums, welches die Resistenz gegen endogene und exogene Faktoren im menschlichen Empfänger erhöht, welches die Blutentnahme bei menschlichen Spendern, Inkubation und Trennung unter anschließender Konservierung des Serums vorsieht, **dadurch gekennzeichnet**, daß man die Spender vor der Blutentnahme der Einwirkung der endogenen oder exogenen Faktoren aussetzt, wobei der Spender für ein sexuell anregendes Serum einer 10-15 min sexuellen Anregung unterworfen wird, für ein somnogenes Serum 20-24 Stunden schlaflos gehalten wird, für ein ophthalmologisches Serum einer verminderten Belichtung von 0,2 lx für 45 bis 60 min ausgesetzt wird, für ein myogenaktives Serum einer körperlichen Belastung innerhalb von 10-15 min unterworfen wird, für ein audioaktives Serum in eine schallisolierte Kammer für 45 bis 60 min eingebracht wird, für ein antihypoxisches Serum in einer Unterdruckkammer einem Druck von 666 bis 733 hPa (500 bis 550 Torr) innerhalb von 20 bis 30 min unterworfen wird, für ein die Hitzebeständigkeit des Organismus erhöhendes Serum einer Einwirkung von Hochtemperaturen von 65 bis 75 °C innerhalb von 45 bis 60 min unterworfen wird, für ein die Kältebeständigkeit des Organismus erhöhendes Serum Tieftemperaturen von + 10 bis + 14 °C innerhalb von 45 bis 60 min unterworfen wird, für ein diätaktives Serum die Nahrungsmotivation innerhalb von 24 Stunden vor der Blutentnahme beeinflußt wird, indem entweder für ein den Appetit stimulierendes Serum keine Nahrung aufgenommen wird oder für ein den Appetit hemmendes Serum eine kalorienreiche Diät (21000 bis 25000 kJ (5000 bis 6000 kcal)) aufgenommen wird, für ein antialkoholisches Serum die Alkoholaufnahme bis zum Auftreten des vollen Widerwillens gegen Spirituosen (5 bis 8 Stunden lang) provoziert wird, bzw. für ein antinikotisches Serum einer Nikotinprovokation bis zum Auftreten des starken Widerwillens gegen Rauchen (20 bis 40 min) unterworfen wird.

## Claims

**1.** Process for producing a biologically active human blood serum which increases the resistance to endogenic and exogenic factors in human recipients, which provides for taking blood from human donors, incubation and separation and subsequent storage of the serum, characterised in that the donors are exposed to the effect of the endogenic or exogenic factors before taking blood, wherein the donor is subjected to a 10-15 minute sexual stimulus for sexually stimulating serum, is kept awake for 20-24 hours for a somnogenic serum, is exposed to reduced light exposure of 0.2 lx for 45 to 60 minutes for an ophthalmological serum, is subjected to bodily stress in the course of 10-15 minutes for a myogen-active serum, is introduced into a soundproof chamber for 45 to 60 minutes for an audio-active serum, is subjected in a negative-pressure chamber to a pressure of 666 to 733 hPa (500 to 550 Torr) in the course of 20 to 30 minutes for an anti-hypoxic serum, is subjected to action of high temperatures of 65 to 75°C in the course of 45 to 60 minutes for a serum which increases the heat resistance of the organism, is subjected to low temperatures of +10 to +14°C in the course of 45 to 60 minutes for a serum which increases the cold resistance of the organism, the digestive motivation is influenced in the course of 24 hours before taking blood for a diet-active serum by either no food being taken for an appetite-stimulating serum or a calorie-rich diet (21,000 to 25,000 kJ (5,000 to 6,000 kcal)) being taken for an appetite-inhibiting serum, alcohol intake is provoked until the occurrence of complete aversion to spirits (for 5 to 8 hours) for an anti-alcoholic serum, or is subjected to nicotine provocation until the occurrence of strong aversion to smoking (20 to 40 minutes) for an anti-nicotine serum.

## Revendications

**1.** Procédé de préparation d'un sérum sanguin humain biologiquement actif, qui augmente la résistance envers les facteurs endogènes et exogènes dans des récepteurs humains, qui prévoit le prélèvement de sanguin chez des donneurs humains, l'incubation et la séparation avec mise en conservation subséquente du sérum, caractérisé en ce que l'on expose les donneurs, avant le prélèvement sanguin, à l'effet des facteurs endogènes ou exogènes, sachant que, pour un sérum stimulant sexuellement, le donneur est soumis à une excitation sexuelle de 10-15 mn, pour un sérum somnogène, le donneur est maintenu sans sommeil pendant 20-24 heures, pour un sérum ophtalmologique, le donneur est exposé à une illumination réduite de 0,2 lx pour une durée de 45 à 60 minutes, pour un sérum à activité myogène, le donneur est soumis à une sollicitation corporelle sur une durée de 10-15 minutes, pour un sérum audioactif, le donneur est introduit dans une chambre isolée du bruit pendant une durée de 45 à 60 minutes, pour un sérum antihypoxique, le donneur est soumis, dans une chambre à dépression, à une pression comprise entre 666 et 733 hPa (500 à 550 Torr), dans une plage de temps de 20 à 30 minutes, pour un sérum augmentant la résistance à la chaleur de l'organisme, le donneur est soumis à l'effet des hautes températures de 65 à 75°C sur une durée de 45 à 60 minutes, pour un sérum augmentant la résistance au froid de l'organisme, le donneur est soumis à de basses températures de +10 à +14°C sur une durée de 45 à 60 minutes, pour un sérum diététique, la motivation nutritive sur 24 heures étant influencée avant le prélèvement sanguin, en ce que, soit pour un sérum stimulant l'appétit aucune nourriture n'est effectuée, soit pour un sérum entravant l'appétit, un régime riche en calorie (21000 à 25000 kJ (5000 à 6000 kcal)) est suivi, pour un sérum anti alcoclique, la prise d'alcool, jusqu'à la survenance de la pleine répugnance contre les spiritueux (sur une durée de 5 à 8 heures), est provoquée, respectivement pour un sérum antinicotique, le donneur est soumis à une provocation de nicotine jusqu'à survenance d'une forte répugnance contre le fait de fumer (20 à 40 minutes).